# EUROPÄISCHE PATENTSCHRIFT

(11) **EP 0 799 013 B1**
(45) Veröffentlichungstag und Bekanntmachung des Hinweises auf die Patenterteilung: **30.09.1998**
(21) Anmeldenummer: 95943202.2
(22) Anmeldetag: 22.12.1995
(51) Int. Cl.: A61J 3/10, B30B 11/16, A61K 9/20

(54) **VERFAHREN ZUR HERSTELLUNG VON TEILBAREN TABLETTEN**
METHOD OF MANUFACTURING DIVISIBLE TABLETS
PROCEDE DE FABRICATION DE COMPRIMES SECABLES

(30) Priorität: 23.12.1994 DE 4446470
(43) Veröffentlichungstag der Anmeldung: 08.10.1997
(73) Patentinhaber: BASF AKTIENGESELLSCHAFT, 67056 Ludwigshafen (DE)
(72) Erfinder: ROSENBERG, Jörg, D-67158 Ellerstadt (DE); MAIER, Werner, D-67105 Schifferstadt (DE); FRICKE, Helmut, D-67112 Mutterstadt (DE); BREITENBACH, Jörg, D-68199 Mannheim (DE)
(74) Vertreter: Kinzebach, Werner, Dr.
(86) Internationale Anmeldenummer: EP9505117
(87) Internationale Veröffentlichungsnummer: WO9619962

(56) Entgegenhaltungen:
- CH-A- 683 066
- DE-A- 1 766 546
- DE-C- 232 863
- DE-C- 395 345
- US-A- 4 880 585

## Beschreibung

Die vorliegende Erfindung betrifft ein Verfahren zur Herstellung von teilbaren Tabletten durch Formen einer wirkstoffhaltigen Schmelze in einem Kalander mit gegenläufig rotierenden Formwalzen, die an ihrer Oberfläche Vertiefungen zur Aufnahme und Formung der Tablettenmasse aufweisen (Schmelzkalandrierung).

Die Herstellung von Tabletten durch Kalandrierung einer wirkstoffhaltigen Schmelze ist aus der DE-A- 1 766 546 und der US-A-4,880,585 bekannt. Grundlage dieses Verfahren ist die Einbettung eines Wirkstoffes in eine Schmelze aus einem Träger, z.B. Fettsubstanzen oder wasserlösliche, thermoplastische Polymere. Die Schmelze wird dadurch erzeugt, daß die Mischung aus Wirkstoff, Polymer und gegebenenfalls weiteren Hilfsstoffen beispielsweise in einem Extruder aufgeschmolzen und als Schmelze in einem nachgeschalteten Formkalander zu Tabletten geformt wird, die durch Abkühlen aushärten. Der Formkalander umfaßt ein sich gegenläufig drehendes Formwalzenpaar, wobei die Formwalzen auf ihrer Oberfläche korrespondierende Gravuren (Vertiefungen) aufweisen, die der Form einer Hälfte der gewünschten Tablette entsprechen. Die Tablettenformung erfolgt im Berührungsbereich der beiden Walzen durch Kombination der Tablettenmasse einer Vertiefung auf der einen Walze mit derjenigen der gegenüberliegenden Vertiefung auf der anderen Walze. Die Kalandrierung der wirkstoffhaltigen Schmelze gemäß US-A-4,880,585 erfolgte mit einem Formwalzenpaar mit identischen Vertiefungen. Auf diese Weise liefert jede Formwalze eine identisch geformte Tablettenhälte, so daß die erhaltenen Tabletten symmetrisch sind.

Häufig ist es erwünscht, daß Tabletten teilbar sind, um die Dosierung variieren zu können, ohne daß für bestimmte Dosierungen jeweils eigene Tabletten hergestellt werden müssen. Bei der Kalandrierung stieß die Herstellung von teilbaren Tabletten jedoch auf erhebliche Schwierigkeiten. Man hat versucht, die Formwalzen mit Vertiefungen zu versehen, die Tabletten liefern, welche den nach konventionellen Tablettiertechnologien hergestellten teilbaren Tabletten optisch gleichen. Dies wurde dadurch bewerkstelligt, daß beim Ausfräsen der Vertiefungen in der Mitte des Bodens jeder Vertiefung eine kleine, oft im Mikrometerbereich liegende Rippe ausgespart blieb, die zur Ausbildung der sogenannten Bruchrille (beidseitig auf jeder Tablettenhälfte) in den fertiggestellten Tabletten führt. Die Anfertigung derartiger Formwalzen ist aber mit erheblichen Mehrkosten verbunden. Nach dem Ausfräsen der Vertiefungen ist nämlich eine Polierung zur Oberflächenglättung erforderlich. Dies kann in einem derartigen Fall nur manuell erfolgen und stößt wegen der in den Vertiefungen vorhandenen Rippen auf erhebliche Schwierigkeiten.

Der vorliegenden Erfindung liegt daher die Aufgabe zugrunde, ein Verfahren zur Herstellung teilbarer Tabletten durch Schmelzkalandrierung zur Verfügung zu stellen, das auf einfache und kostensparende Weise durchführbar ist.

Überraschenderweise wurde nun gefunden, daß diese Aufgabe gelöst werden kann, wenn man zwei Formwalzen miteinander kombiniert, von denen mindestens eine Vertiefungen aufweist, die durch mindestens einen Steg voneinander getrennt sind, der sich im wesentlichen bis zur Mantellinie erstreckt.

Gegenstand der vorliegenden Erfindung ist daher ein Verfahren zur Herstellung von teilbaren Tabletten durch Formen einer wirkstoffhaltigen Schmelze in einem Kalander mit zwei gegenläufig rotierenden Formwalzen, die Vertiefungen zur Aufnahme und Formung der Schmelze zu Tabletten aufweisen, das dadurch gekennzeichnet ist, daß man mindestens eine Formwalze verwendet, bei der die Vertiefungen durch mindestens einen Steg unterteilt sind, der sich im wesentlichen bis zur Mantelfläche der Formwalze erstreckt und die Formung einer Bruchrille bewirkt.

Die Tabletten werden durch Kombination der Vertiefungen auf der ersten Formwalze mit den entsprechenden Vertiefungen auf der zweiten Formwalze gebildet. Der in den Vertiefungen vorhandene Steg ist relativ schmal und führt beim Formen der Tabletten zur Bildung der Bruchrille.

Gemäß einer bevorzugten Ausführungsform verwendet man eine erste Formwalze, bei der die Vertiefungen durch n Stege unterteilt sind und eine zweite Formwalze mit entsprechenden Vertiefungen, die durch n' Stege unterteilt sind. Dabei liegt n' im Bereich von 0 bis n-1.

Die Zahl n der Stege in den Vertiefungen auf der ersten Walze richtet sich danach, in wieviel Teile die Tablette teilbar sein soll. Im einfachsten und häufigsten Fall wird diese Zahl eins sein (n = 1). Wenn man eine damit erhaltene Tablettenhälfte kombiniert mit einer Tablettenhälfte, die resultiert aus einer Vertiefung mit n' = n-1 = 0, also keinem Steg, auf der zweiten Walze, erhält man eine Tablette, die in zwei Teile teilbar ist. Üblicherweise teilt der Steg die Vertiefungen auf der ersten Walze in zwei gleiche Teile (spiegelbildlich im wesentlichen identisch), so daß man eine Tablette erhält, die in zwei gleiche Teile teilbar ist. Die beiden Teile einer Vertiefung auf der ersten Walze können aber auch verschieden sein, d.h. man erhält dann eine Tablette, die in zwei unterschiedliche Teile teilbar ist, beispielsweise kann der eine Teil 1/3 und der andere 2/3 der Tablette ausmachen, so daß auch 1/3 bzw. 2/3 der in der Tablette enthaltenen Wirkstoffmenge bequem verabreicht werden kann. Diese Ausführungsform mit n=1 und n'=0 ist besonders vorteilhaft zur Herstellung teilbarer Oblong-Tabletten und linsenförmiger Tabletten.

Die Zahl der Stege n' in einer Vertiefung auf der zweiten Formwalze beträgt höchstens n-1. Diese Zahl kann aber auch geringer sein und sie liegt daher im Bereich von 0 bis n-1. Beispielsweise kann man Vertiefungen mit zwei Stegen (n=2) auf der ersten Formwalze zur Kombination mit entsprechenden Vertiefungen ohne Steg oder mit einem Steg auf der zweiten Formwalze (n'=0 oder 1) vorsehen. Man erhält dann eine Tablette, die sich dritteln läßt, oder eine Tablette, die sich sowohl dritteln als auch (n=2; n'=0) halbieren läßt (n=2; n'=1).

Je nach Anpreßdruck der Formwalzen, kann man eine "Kette" von 2, 3 oder mehreren Tabletten, von der eine beliebige Anzahl abgenommen werden kann, um die Dosierung zu variieren, oder (bei höherem Anpreßdruck) einzelne teilbare Tabletten herstellen.

Gemäß einer besonders bevorzugten Ausführungsform ist die zweite Formwalze eine Glattwalze, d.h. sie weist keine Vertiefungen auf. Auf diese Weise läßt sich bei gegebener Wirkstoffkonzentration in der Schmelze durch Variation der Zahl der Stege in den Vertiefungen auf der ersten Walze eine weitgehende Variation der Dosierung bewerkstelligen. Um teilbare Tabletten zu erhalten, endet der Steg entweder knapp unterhalb der Mantelfläche der Formwalze oder der Anpreßdruck zwischen den beiden Formwalzen ist relativ gering oder es besteht sogar ein geringer Abstand zwischen den Formwalzen (z.B. 0,1 - 1 mm), wenn sich der Steg bis zur Mantelfläche erstreckt.

Diese Ausführungsform hat zudem Vorteile gegenüber allen anderen, die auf einer Kombination von zwei Formwalzen mit Vertiefungen beruhen. Bei der Tablettenherstellung über zwei Formwalzen mit Vertiefungen müssen diese jeweils einander exakt gegenüberliegen, damit die gebildeten Tablettenhälften nicht gegeneinander versetzt vorliegen, was hohe Anforderungen an die Präzision bei der Herstellung der Vertiefungen und bei der Rotation der Formwalzen stellt. Das Problem des Versatzes tritt bei einer Kombination mit einer glatten Formwalze dagegen nicht auf, weil hier keine zwei Tablettenhälften existieren, die zueinander versetzt vorliegen könnten. Diese Ausführungsform bietet sich daher vor allem bei kleineren Tablettenformen an, bei denen sich schon sehr kleine Ungenauigkeiten der Kalander-Formwalzenführung stark auf das Erscheinungsbild der Tabletten auswirken.

Die Herstellung der Tabletten erfolgt ausgehend von einer Mischung, die einen oder mehrere pharmazeutische Wirkstoffe sowie einen oder mehrere übliche Hilfsstoffe enthält und die durch Schmelzen oder Erweichen mindestens einer Komponente teigig bis zähflüssig und daher extrudierbar wird.

Das sind insbesondere Mischungen die pharmakologisch akzeptable Polymere enthalten (wobei die Glastemperatur der Mischung unter der Zersetzungstemperatur aller Mischungskomponenten liegt), beispielsweise Polyvinylpyrrolidon (PVP), Copolymerisate von N-Vinylpyrrolidon (NVP) und Vinylacetat, Copolymerisate von Vinylacetat und Crotonsäure, teilverseiftes Polyvinylacetat, Polyvinylalkohol, Ethylen/Vinylacetat-Copolymerisate, Polyhydroxyethylmethacrylat, Copolymerisate von Methylmethacrylat und Acrylsäure, Celluloseester, Celluloseether, Polyethylenglykol oder Polyethylen, bevorzugt NVP-Copolymerisate mit Vinylacetat, Hydroxypropylcellulose und Polyethylenglycole/Polyethylenoxide. Die K-Werte (nach H. Fikentscher, Cellulose-Chemie 13 (1932), Seiten 58 bis 64 und 71 und 74) der Polymeren liegen im Bereich von 10 bis 100, vorzugsweise 12 bis 70, insbesondere 12 bis 35, für PVP vorzugsweise bei 12 bis 35, insbesondere bei 12 bis 17.

Das polymere Bindemittel muß in der Gesamtmischung aller Komponenten im Bereich von 50 bis 180, vorzugsweise 60 bis 130°C erweichen oder schmelzen, so daß die Masse extrudierbar ist. Die Glasübergangstemperatur der Mischung muß also auf jeden Fall unter 180, vorzugsweise unter 130°C liegen. Erforderlichenfalls wird sie durch übliche pharmakologisch akzeptable weichmachende Hilfsstoffe wie langkettige Alkohole, Ethylenglykol, Propylenglykol, Trimethylolpropan, Triethylenglykol, Butandiole, Pentanole, Hexanole, Polyethylenglykole, Silicone, aromatische Carbonsäureester (z.B. Dialkylphthalate, Trimellithsäureester, Benzoesäureester, Terephthalsäureester) oder aliphatische Dicarbonsäureester (z.B. Dialkyladipate, Sebacinsäureester, Azelainsäureester, Zitronen- und Weinsäureester) oder Fettsäureester herabgesetzt.

Übliche galenische Hilfsstoffe, deren Gesamtmenge bis zu 100 Gew.-% bezogen auf das Polymerisat, betragen kann, sind z.B. Streckmittel wie Silikate oder Kieselerde, Stearinsäure oder deren Salze, z.B. das Magnesium- oder Kalziumsalz, Methylcellulose, Natrium-Carboxymethylcellulose, Talkum, Saccharose, Lactose, Getreide- oder Maisstärke, Kartoffelmehl, Polyvinylalkohol, ferner Netz-, Konservierungs-, Spreng-, Adsorptionsmittel, Farbstoffe, Geschmacksstoffe (vgl. z.B. H. Sucker et al. Pharmazeutische Technologie, Thieme-Verlag, Stuttgart 19/8).

Unter pharmazeutischen Wirkstoffen im Sinne der Erfindung sind alle Stoffe mit einer pharmazeutischen Wirkung und möglichst geringen Nebenwirkungen zu verstehen, sofern sie sich unter den Verarbeitungsbedingungen nicht zersetzen. Die Wirkstoffmenge pro Dosiseinheit und die Konzentration können je nach Wirksamkeit und Freisetzungsgeschwindigkeit in weiten Grenzen variieren. Die einzige Bedingung ist, daS sie zur Erzielung der gewünschten Wirkung ausreichen. So kann die Wirkstoffkonzentration im Bereich von 0,1 bis 95, vorzugsweise von 20 bis 80, insbesondere 30 bis 70 Gew.-% liegen. Auch Wirkstoff-Kombinationen können eingesetzt werden. Wirkstoffe im Sinne der Erfindung sind auch Vitamine und Mineralstoffe sowie Pflanzenbehandlungsmittel und Insektizide.

Das erfindungsgemäße Verfahren ist beispielsweise zur Verarbeitung folgender Wirkstoffe geeignet:

Acebutolol, Acetylcystein, Acetylsalicylsäure, Acyclovir, Albrazolam, Alfacalcidol, Allantoin, Allopurinol, Ambroxol, Amikacin, Amilorid, Aminoessigsäure, Amiodaron, Amitriptylin, Amlodipin, Amoxicillin, Ampicillin, Ascorbinsäure, Aspartam, Astemizol, Atenolol, Beclomethason, Benserazid, Benzalkonium Hydroxid, Benzocain, Benzoesäure, Betamethason, Bezafibrat, Biotin, Biperiden, Bisoprolol, Prazosin, Bromazepam, Bromhexin, Bromocriptin, Budesonid, Bufexamac, Buflomedil, Buspiron, Coffein, Campher, Captopril, Carbamazepin, Carbidopa, Carboplatin, Carotinoide wie beispielsweise β-Carotin oder Canthaxanthin, Cefachlor, Cefalexin, Cefatroxil, Cefazolin, Cefixim, Cefotaxim, Ceftazidim, Ceftriaxon, Cefuroxim, Celedilin, Chloramphenicol, Chlorhexidin, Chlorpheniramin, Chlortalidon, Cholin, Cyclosporin, Cilastatin, Cimetidin, Ciprofloxacin, Cisapride, Cisplatin, Clarithromycin, Clävulansäure, Clomibramin, Clonazepam, Clonidin, Clotrimazol, Codein, Cholestyramin, Cromoglycinsäure, Cyanocobalamin, Cyproteron, Desogestrel, Dexamethason, Dexpanthenol, Dextromethorphan, Dextropropoxiphen, Diazepam, Diclofenac, Digoxin, Dihydrocodein, Dihydroergotamin, Diltiazem, Diphenhydramin, Dipyridamol, Dipyron, Disopyramid, Domperidon, Dopamin, Enalapril, Ephedrin, Epinephrin, Ergocalciferol, Ergotamin, Erythromycin, Estradiol, Ethinylestradiol, Etoposide, Eucalyptus Globulus, Famotidin, Felodipin, Fenofibrat, Fenoterol, Fentanyl, Flavin-Mononucleotid, Fluconazol, Flunarizin, Fluorouracil, Fluoxetin, Flurbiprofen, Furosemid, Gemfibrozil, Gentamicin, Ginkgo Biloba, Glibenclamid, Glipizid, Clozapin, Glycyrrhiza glabra, Guaifenesin, Haloperidol, Heparin, Hyaluronsäure, Hydrochlorothiazid, Hydrocodon, Hydrocortison, Hydromorphon, Ipratropium Hydroxid, Ibuprofen, Imipenem, Indomethacin, Iohexol, Iopamidol, Isosorbid-Dinitrat, Isosorbid-Mononitrat, Isotretinoin, Ketotifen, Ketoconazol, Ketoprofen, Ketorolac, Labatalon, Lactulose, Lecithin, Levocarnitin, Levodopa, Levoglutamide, Levonorgestrel, Levothyroxin, Lidocain, Lipase, Liponsäure, Lisinopril, Loperamid, Lorazepam, Lovastatin, Medroxyprogesteron, Menthol, Methotrexat, Methyldopa, Methylprednisolon, Metoclopramid, Metoprolol, Miconazol, Midazolam, Minocyclin, Minoxidil, Misoprostol, Morphin, Multivitamin-Mischungen bzw. -kombinationen und Mineralsalze, N-Methylephedrin, Naftidrofuryl, Naproxen, Neomycin, Nicardipin, Nicergolin, Nicotinamid, Nicotin, Nicotinsäure, Nifedipin, Nimodipin, Nitrendipin, Nizatidin, Norethisteron, Norfloxacin, Norgestrel, Nortriptylin, Nystatin, Ofloxacin, Omeprazol, Ondanseron, Pancreatin, Panthenol, Pantothensäure, Paracetamol, Penicillin G, Penicillin V, Phenobarbital, Phenoxifyllin, Phenylephrin, Phenylpropanolamin, Phenytoin, Piroxicam, Polymyxin B, Povidone-Iod, Pravastatin, Prednisolon, Bromocriptin, Propafenon, Propranolol, Pseudoephedrin, Pyridoxin, Quinidin, Ramipril, Ranitidin, Reserpin, Retinol, Riboflavin, Rifampicin, Rutosid, Saccharin, Salbutamol, Salcatonin, Salicylsäure, Simvastatin, Somatropin, Sotalol, Spironolacton, Sucralfat, Sulbactam, Sulfamethoxazol, Sulpiridid, Tamoxifen, Tegafur, Teprenon, Terazosin, Terbutalin, Terfenadin, Theophyllin, Thiamin, Tiolopidin, Timolol, Tranexamsäure, Tretinoin, Triamcinolon-Acetonid, Triamteren, Trimethoprim, Troxerutin, Uracil, Valproinsäure, Vancomycin, Verapamil, Vitamin B₁, B₂, B₄, B₆, B₁₂, D₃, E, K, Volinsäure, Zidovudin.

In Einzelfällen kann es zur Ausbildung fester Lösungen kommen. Der Begriff "feste Lösungen" ist dem Fachmann geläufig, beispielsweise aus der eingangs zitierten Literatur. In festen Lösungen von pharmazeutischen Wirkstoffen in Polymeren liegt der Wirkstoff molekulardispers im Polymer vor.

Die pharmazeutische Mischung wird dann in üblicher Weise aufgeschmolzen, vorzugsweise in einem Extruder, und dem Formkalander zugeführt, wie das beispielsweise in der US-A-4,880,585 beschrieben ist. Falls erforderlich werden die Tabletten nach der Kalandrierung gekühlt, z.B. in einem Luft- oder Kühlbad.

Bei klebrigen oder hochviskosen Materialien, die sich nur schwer oder gar nicht von der Form lösen, ist die Anwendung eines Formentrennmittels, beispielsweise ein Silikonöl, Silikonlack, Triglycerid oder Lecithin, zweckmäßig.

Gewünschtenfalls können die Tabletten mit einer Umhüllung versehen werden, insbesondere um den Geschmack zu maskieren oder die Tabletten durch Farbgebung unterscheidbar zu machen oder zu verpacken. Zu diesem Zweck führt man die wirkstoffhaltige Schmelze zwischen zwei Folien aus dem Umhüllungsmaterial in die Formwalzen ein.

Das Umhüllungsmaterial kann aus einer breiten Palette von Materialien ausgewählt werden. Voraussetzung ist lediglich, daß es sich um ein pharmazeutisch akzeptables Material handelt.

Für die Herstellung von Filmtabletten geeignete Umhüllungsmaterialien, die sich rasch im sauren Magensaft auflösen sind Folien aus z.B. Gelatine, Polyvinylalkohol, Alkylcellulosen, wie Methylcellulosen, Hydroxyalkylcellulosen, wie Hydroxyethyl-, Hydroxypropyl- oder Hydroxypropylmethylcellulose, Polyvinylpyrrolidon, bestimmte Acrylharze, wie Copolymerisate auf Basis von Dimethylaminoethylmethacrylat und Methacrylestern (Eudragit E) etc.

Das Umhüllungsmaterial kann gewünschtenfalls einen Farbstoff oder ein Pigment oder auch einen weiteren Wirkstoff enthalten.

Gemäß einer weiteren Ausführungsform verwendet man als Folien solche, die für eine Verpackung der Tabletten geeignet sind. Es handelt sich dabei insbesondere um wasserunlösliche Tiefziehfolien, wobei als Material Polyethylen, Polypropylen, Polyvinylchlorid, Polyethylenterephthalat, Polystyrol, Aluminium oder beschichtetes Aluminium bevorzugt ist. Auf diese Weise werden die Tabletten sofort in eine Blisterpackung eingesiegelt. Der sonst übliche eigene Verpackungsschritt entfällt somit und außerdem ist es auf diese Weise möglich, die Tabletten auf einfachste Weise aseptisch zu verpacken, insbesondere dann, wenn man dafür Sorge trägt, daß die Außenkanten des Tablettenbandes luftdicht verschweißt werden.

Es hat sich gezeigt, daß nicht, wie erwartet, eine intensive Verklebung der heißen Tablettenmasse mit der wasserunlöslichen Tiefziehfolie erfolgt, so daß die spätere Entnahme der Tabletten aus der Verpackung behindert oder sogar unmöglich gewesen wäre.

Für die Verpackung der Tabletten hat es sich als besonders vorteilhaft erwiesen, eine Formwalze mit den Vertiefungen für die Aufnahme und Formung der Tablettenmasse mit einer Glattwalze zu kombinieren. Man erhält auf diese Weise "Halb"-Tabletten, die in eine Blisterpackung eingesiegelt sind, welche auf einer Seite Vertiefungen für die Aufnahme der Tabletten aufweist und auf der anderen Seite mit einer glatten, abziehbaren Folie verschlossen ist. In diesem Fall hat sich eine Aluminiumfolie oder eine Folie aus beschichtetem Aluminium für den Verschluß der Verpackung als besonders zweckmäßig erwiesen.

Es kann sich als zweckmäßig erweisen, die verpackten Tabletten nicht, wie sonst üblich, an der Luft abkühlen zu lassen sondern einen eigenen Abkühlungsschritt vorzusehen. Hierfür ist ein Wasserbad, kalter Luftstrom etc. geeignet. Auf diese Weise wird vermieden, daß die Tabletten in der Verpackung zu langsam erkalten, was zu nachträglichen Verformungen der Tabletten führen kann.

Es ist auch möglich, die Folien für den Filmüberzug der Tabletten und die Folien für die Verblisterung der Tabletten gleichzeitig zu verwenden. In diesem Fall wird die Schmelze in den Formwalzen von der Folie für den Filmüberzug umhüllt und gleichzeitig in die Verpackungsfolie eingesiegelt.

Die Form der Vertiefungen und damit der erfindungsgemäß erhältlichen Tabletten kann weitgehend beliebig gewählt werden. Neben den schon erwähnten Oblong-Tabletten haben sich linsenförmige Tabletten als besonders zweckmäßig erwiesen. Die für ihre Herstellung verwendeten Formwalzen haben ellipsoidsegmentartige, insbesondere kugelsegmentartige Vertiefungen. Der Winkel zwischen der Tangentialfläche der Vertiefungen am oberen Rand und er Tangentialfläche der Formwalze (im Schnittpunkt der Vertiefung mit der Walzenoberfläche) ist dabei < 90°, vorzugsweise < 45°. Die linsenförmigen Tabletten haben den Vorteil, daß sie sich besonders leicht entgraten lassen.

Gegenstand der vorliegenden Erfindung ist auch eine Vorrichtung (Kalander) zur Durchführung des erfindungsgemäßen Verfahrens mit zwei gegenläufig rotierbaren Formwalzen, die sich ggf. entlang einer Mantellinie berühren und Vertiefungen zur Aufnahme und Formung der Schmelze zu Tabletten aufweisen, die dadurch gekennzeichnet ist, daß mindestens eine Formwalze Vertiefungen aufweist, die durch mindestens einen Steg unterteilt sind, der sich im wesentlichen bis zur Mantelfläche der Formwalze erstreckt und die Formung einer Bruchrille bewirkt.

Im allgemeinen erstreckt sich der erwähnte Steg bis zur Mantelfläche der Formwalze. In manchen Fällen kann es aber zweckmäßig sein, wenn sich der Steg nicht vollständig bis zur Mantellinie erstreckt, d.h. er endet knapp unterhalb der Mantellinie. Dies erhöht die Stabilität der erhaltenen Tablette.

Gemäß einer bevorzugten Ausführungsform weist die erste Formwalze Vertiefungen auf, die durch n Stege unterteilt sind, während die entsprechenden Vertiefungen auf der zweiten Formwalze durch n' Stege unterteilt sind, wobei n' im Bereich von 0 bis n-1 liegt.

Zweckmäßigerweise sind die Vertiefungen auf der zweiten Formwalze tiefer als diejenigen auf der ersten Formwalze. Man erhält dann asymmetrische Tabletten, bei denen die "Hälfte" ohne Bruchrille größer ist als die "Hälfte" mit Bruchrille. Bei derartigen Tabletten ist die Gefahr des Zerbrechens bei der Konfektionierung oder bei unsachgemäßer Handhabung erheblich geringer.

Vorzugsweise kombiniert man eine erste Formwalze, die Vertiefungen mit einem Steg aufweist mit einer zweiten Formwalze, die eine entsprechende Vertiefung ohne Steg aufweist.

Gemäß einer weiteren Ausführungsform kombiniert man eine erste Formwalze, die Vertiefungen mit zwei Stegen (n=2) aufweist, mit einer zweiten Formwalze, die entsprechende Vertiefungen ohne Steg (n'=0) oder mit einem Steg (n'=1) aufweist.

Gemäß einer besonders bevorzugten Ausführungsform kombiniert man eine erste Formwalze, die Vertiefungen mit mindestens einem Steg aufweist, mit einer Glattwalze.

Die Erfindung wird nachfolgend anhand der Zeichnung und der Beispiele näher erläutert.

### KURZBESCHREIBUNG DER FIGUREN:

Figur 1 zeigt einen Querschnitt durch eine Formwalze nach dem Stand der Technik zur Herstellung von teilbaren Tabletten.

Figur 2 zeigt einen Schnitt durch Oblong-Tabletten, die mit der Formwalze gemäß Figur 1 erhalten wurden.

Figur 3 zeigt einen Querschnitt durch ein Formwalzenpaar gemäß der vorliegenden Erfindung.

Figur 4 zeigt einen Schnitt durch Tabletten, die mit dem Formwalzenpaar gemäß Figur 3 erhalten wurden.

Figur 5 zeigt einen Schnitt durch Tabletten mit einem Versatz zwischen den beiden Tablettenhälften.

Figur 6 zeigt eine erfindungsgemäße Kombination einer Walze mit Vertiefungen mit einer Glattwalze.

Figur 7 zeigt einen Schnitt durch Tabletten, die durch die Walzenkombination der Figur 6 erhalten wurden.

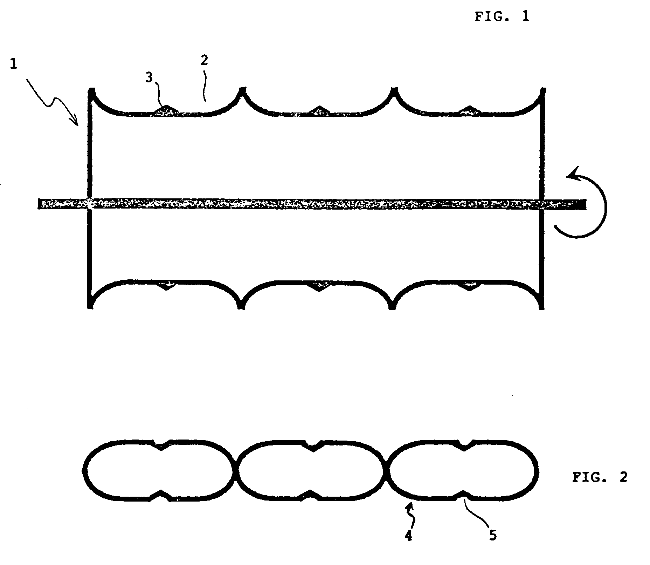

Die Figur 1 zeigt eine Formwalze 1 des Standes der Technik. Sie weist Vertiefungen 2 zur Aufnahme und Formung der geschmolzenen Tablettenmasse zu Tabletten auf. Am Boden dieser Vertiefungen befinden sich quer zur Längsachse der Formwalzen Rippen 3, die beim Ausfräsen der Vertiefungen ausgespart wurden. Wenn man zwei derartige Formwalzen 1 des Standes der Technik miteinander kombiniert und Tabletten durch Schmelzkalandrierung herstellt, erhält man längliche Tabletten 4, sogenannte Oblong-Tabletten der in Figur 2 gezeigten Art. Diese Tabletten weisen eine Bruchrille 5 auf, die es erlaubt, die Tablette in zwei gleiche Teile zu zerbrechen.

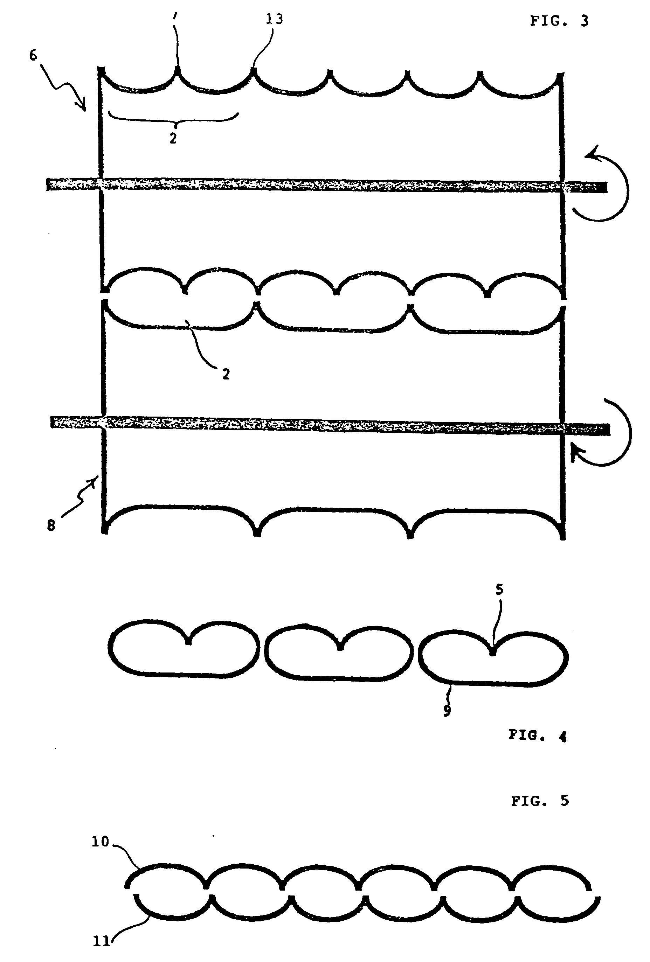

Die Figur 3 zeigt eine erfindungsgemäße Kombination einer ersten Formwalze 6 mit einer zweiten Formwalze 8. Die Formwalze 6 weist drei gleiche, unmittelbar aneinandergrenzende Vertiefungen 2 auf. Diese Vertiefungen sind jeweils durch einen Steg 7 unterteilt, der bis zur Mantelfläche der Formwalze 6 reicht. Die zweite Formwalze 8 besitzt ebenfalls drei unmittelbar aneinandergrenzende, in der Form entsprechende Vertiefungen 2. Alle Vertiefungen 2 sind im gezeigten Fall nur durch einen schmalen Grat 13 voneinander getrennt, der im gezeigten Fall im wesentlichen dem Steg 7 entspricht. Zwischen den zur Bildung einer Tablette verwendeten Vertiefungen 2 kann jedoch auch ein größerer Zwischenraum, d.h. ein breiterer Grat 13 liegen.

Die Umfangslinie um die Vertiefungen 2 der Formwalze 6 (in der Mantelfläche der Formwalze) entspricht der Umfangslinie um die Vertiefungen der Formwalze 8, d.h. die Grundflächen der erhaltenen Tablettenhälften entsprechen sich, so daß die beiden Hälften zu einer Tablette zusammengefügt werden können.

Man erhält auf diese Weise teilbare Tabletten 9 der in Figur 4 gezeigten Art. Sie weisen eine Bruchlinie 5 auf, die es ermöglicht, die Tabletten in zwei gleiche Hälften zu teilen.

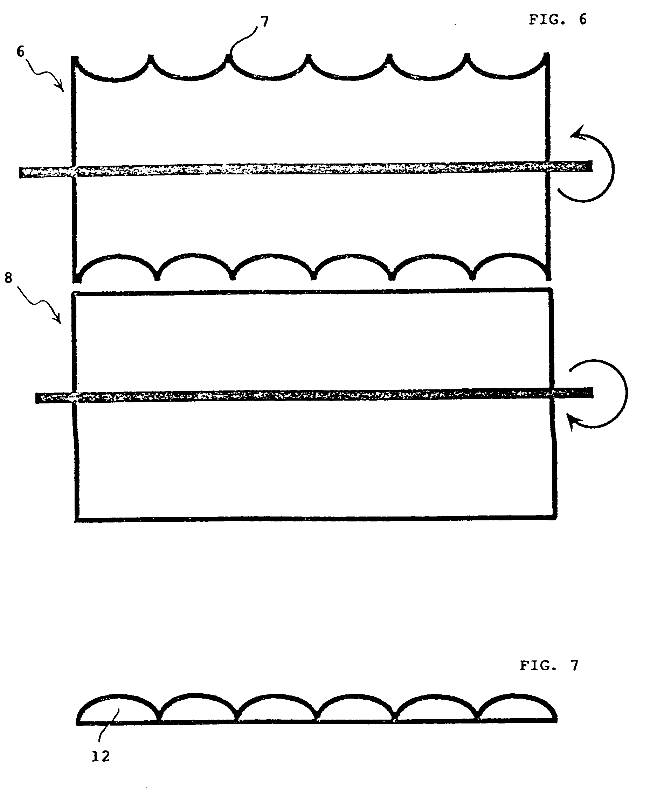

Wie oben erwähnt, muß die Präzision bei der Herstellung und Rotation der Formwalzen sehr hoch sein, um zu vermeiden, daß die obere Tablettenhälfte gegen die untere versetzt ist. Ein derartiger Versatz zwischen den Tablettenhälften 10 und 11 ist in Figur 5 gezeigt.
Mit der in Figur 6 gezeigten Kombination aus einer ersten Formwalze 6 mit fünf Stegen 7 mit einer als Glattwalze ausgebildeten zweiten Formwalze 8 wird ein derartiger Versatz zwischen den Tablettenhälften vermieden. Durch die in Figur 6 gezeigte Kombination der Formwalzen erhält man bei relativ geringerem Anpreßdruck oder bei geringem Abstand zwischen den Formwalzen eine "Kette" von "Halb-Tabletten" 12 der in Figur 7 gezeigten Art. Insbesondere diese Art von Tabletten kann in sehr kleinen Abmessungen hergestellt werden, so daß man durch die Wahl der Größe der Vertiefungen und der Länge der "Kette" der Tabletten die Dosierung in weitem Bereich variieren kann.

### BEISPIEL 1

Eine Mischung, bestehend aus 60,0 Gew.-% Kollidon VA-64 (BASF) (Polyvinylpyrrolidon-Copolymerisat mit Vinylacetat (60:40)) und 40,0 Gew.-% Lactose-Monohydrat, wurde in einem Zweischnecken-Extruder (ZSK-40, Fa. Werner + Pfleiderer) unter folgenden Bedingungen extrudiert:

| | |
|---|---|
| - Temperaturen: | |
| Schuß 1: | 80°C |
| Schuß 2: | 100°C |
| Schuß 3: | 130°C |
| Schuß 4: | 130°C |
| Düsen: | 135°C |
| - Materialdurchsatz: | 25 kg/h |
| - Schneckendrehzahl: | 160 U/min |

Die Schmelze wurde in einen Formkalander mit zwei Formwalzen der in Figur 3 gezeigten Art geführt. Man erhielt auf diese Weise Tabletten, wie sie in Figur 4 abgebildet sind. Sie waren leicht und glatt in zwei gleiche Hälften zu zerbrechen.

In entsprechender Weise wurden Tabletten der in Figur 6 gezeigten Kombination von Formwalzen hergestellt. Man erhielt auf diese Weise Tabletten, wie sie in Figur 7 abgebildet sind.

Die im Rahmen der Erfindung verwendbaren Kalander- und Formwalzen können in an sich bekannter Weise gekühlt oder geheizt werden, und die für den jeweiligen Verarbeitungsprozeß optimale Oberflächentemperatur der Formwalze kann auf diese Weise eingestellt werden.

## Patentansprüche

1. Verfahren zur Herstellung von teilbaren Tabletten durch Formen einer wirkstoffhaltigen Schmelze in einem Kalander mit zwei gegenläufig rotierenden Formwalzen, die Vertiefungen zur Aufnahme und Formung der Schmelze zu Tabletten aufweisen, **dadurch gekennzeichnet,** daß man mindestens eine Formwalze verwendet, bei der die Vertiefungen durch mindestens einen Steg unterteilt sind, der sich im wesentlichen bis zur Mantelfläche der Formwalze erstreckt und die Formung einer Bruchrille bewirkt.

2. Verfahren nach Anspruch 1, dadurch gekennzeichnet, daß man eine erste Formwalze verwendet, bei der die Vertiefungen durch n Stege unterteilt sind und eine zweite Formwalze mit entsprechenden Vertiefungen, die durch n' Stege unterteilt sind, wobei n' im Bereich von 0 bis n-1 liegt.

3. Verfahren nach Anspruch 2, dadurch gekennzeichnet, daß man eine erste Formwalze, die Vertiefungen mit einem Steg (n=1) aufweist und eine zweite Formwalze verwendet, die entsprechende Vertiefungen ohne Steg (n'=0) aufweist.

4. Verfahren nach Anspruch 2, dadurch gekennzeichnet, daß man eine erste Formwalze, die Vertiefungen mit zwei Stegen (n=2) aufweist und eine zweite Formwalze verwendet, die Vertiefungen mit einem Steg (n'=1) aufweist.

5. Verfahren nach Anspruch 1, dadurch gekennzeichnet, daß man eine erste Formwalze verwendet, welche Vertiefungen mit mindestens einem Steg (n ≥ 1) aufweist und als zweite Formwalze eine Glattwalze verwendet.

6. Verfahren nach einem der vorhergehenden Ansprüche, dadurch gekennzeichnet, daß man die wirkstoffhaltige Schmelze zwischen zwei Folien aus einem Umhüllungsmaterial in die Formwalzen einführt, so daß man Filmtabletten oder in eine Blisterpackung eingesiegelte Tabletten erhält.

7. Verfahren nach einem der vorhergehenden Ansprüche, dadurch gekennzeichnet, daß man Formwalzen mit länglichen oder ellipsoidsegmentartigen Vertiefungen verwendet, so daß man teilbare Oblong-Tabletten oder linsenförmige Tabletten erhält.

8. Vorrichtung zur Durchführung des Verfahrens der Ansprüche 1 bis 7 mit zwei gegenläufig rotierbaren Formwalzen (6,8), die Vertiefungen (2) zur Aufnahme und Formung der Schmelze zu Tabletten aufweisen, dadurch gekennzeichnet, daß mindestens eine der Formwalzen Vertiefungen (2) aufweist, die durch mindestens einen Steg (7) unterteilt sind, der sich im wesentlichen bis zur Mantelfläche der Formwalze erstreckt und die Formung einer Bruchrille bewirkt.

9. Vorrichtung nach Anspruch 8, dadurch gekennzeichnet, daß die erste Formwalze (6) Vertiefungen (2) aufweist, die durch n Stege unterteilt sind und die zweite Formwalze (8) entsprechende Vertiefungen (2) aufweist, die durch n' Stege unterteilt sind, wobei n' im Bereich von 0 bis n-1 liegt.

10. Vorrichtung nach Anspruch 9, dadurch gekennzeichnet, daß die erste Formwalze (6) Vertiefungen (2) mit einem Steg (7) (n = 1) aufweist, und die zweite Formwalze (8) entsprechende Vertiefungen (2) ohne Steg (n' = 0) aufweist.

11. Vorrichtung nach Anspruch 9, dadurch gekennzeichnet, daß die erste Formwalze (6) Vertiefungen (2) mit zwei Stegen (7) (n = 2) aufweist und die zweite Formwalze (8) entsprechende Vertiefungen (2) mit einem Steg (7) aufweist.

12. Vorrichtung nach Anspruch 8, dadurch gekennzeichnet, daß die erste Formwalze (6) Vertiefungen (2) mit mindestens einem Steg (7) (n ≥ 1) aufweist und die zweite Formwalze eine Glattwalze (8) ist.

13. Vorrichtung nach einem der Ansprüche 8 bis 12, dadurch gekennzeichnet, daß man Formwalzen (6,8) mit länglichen oder ellipsoidsegmentartigen Vertiefungen (2) verwendet.

## Claims

1. A process for the production of divisible tablets by molding a melt containing an active ingredient in a calender with two counter rotating molding rolls which have depressions for receiving and molding the melt to tablets, characterised in that at least one molding roll in which the depressions are divided by at least one bar which extends essentially as far as the surface line of the molding roll, and forms a score, is used.

2. A process as claimed in claim 1, characterised in that a first molding roll In which the depressions are divided by n bars, and a second molding roll with corresponding depressions which are divided by n' bars, where n' is in the range from 0 to n 1, is used.

3. A process as claimed in claim 2, characterised in that a first molding roll which has depressions with one bar (n 1), and a second molding roll which has corresponding depressions without bar (n'=0) are used.

4. A process as claimed in claim 2, characterised in that a first molding roll which has depressions with two bars (n 2) and a second molding roll which has depressions with one bar (n'=1) are used.

5. A process as claimed in claim 1, characterised in that a first molding roll which has depressions with at least one bar (n ≥ 1) is used, and a smooth roll is used as second molding roll.

6. A process as claimed in any of the preceding claims, characterised in that the melt containing active ingredient is introduced between two sheets of a covering material into the molding rolls so that film coated tablets or tablets sealed in a blister pack are obtained.

7. A process as claimed in any of the preceding claims, characterised in that molding rolls with depressions which are elongate or in the shape of a segment of an ellipsoid are used so that divisible oblong tablets or lenticular tablets are obtained.

8. An appliance for carrying out the process of claims 1 to 7 with two counter rotating molding rolls (6, 8) which have depressions (2) for receiving and molding the melt to tablets, characterised in that at least one of the molding rolls has depressions (2) which are divided by at least one bar (7) which extends essentially up to the surface of the molding roll and forms a score.

9. An appliance as claimed in claim 8, characterised in that the first molding roll (6) has depressions (2) which are divided by n bars, and the second molding roll (8) has corresponding depressions (2) which are divided by n' bars, where n' is in the range from 0 to n 1.

10. An appliance as claimed in claim 9, characterised in that the first molding roll (6) has depressions (2) with one bar (7) (n = 1), and the second molding roll (8) has corresponding depressions (2) without bar (n' = 0).

11. An appliance as claimed in claim 9, characterised in that the first molding roll (6) has depressions (2) with two bars (7) (n = 2), and the second molding roll (8) has corresponding depressions (2) with one bar (7).

12. An appliance as claimed in claim 8, characterised in that the first molding roll (6) has depressions (2) with at least one bar (7) (n ≥ 1), and the second molding roll is a smooth roll (8).

13. An appliance as claimed in any of claims 8 to 12, characterised in that molding rolls (6, 8) with depressions (2) which are elongate or in the shape of a segment of an ellipsoid are used.

## Revendications

1. Procédé de fabrication de comprimés subdivisables, par moulage d'un produit fondu contenant un principe actif, dans une calandre, avec deux rouleaux de moulage tournant en sens inverses, présentant des cavités destinées à recevoir et à mouler le produit en fusion pour former des comprimés, caractérisé par le fait qu'on utilise au moins un rouleau de moulage, pour lequel les cavités sont subdivisées au moyen d'au moins une nervure qui s'étend sensiblement jusqu'à la surface d'enveloppe du rouleau de moulage, et provoque le moulage d'une cannelure de rupture.

2. Procédé selon la revendication 1, caractérisé par le fait qu'on utilise un premier rouleau de moulage pour lequel les cavités sont subdivisées par n nervures et un deuxième rouleau de moulage présentant des cavités correspondantes, subdivisées par n' nervures, n' étant située dans la plage de 0 à n-1.

3. Procédé selon la revendication 2, caractérisé par le fait qu'on utilise un premier rouleau de moulage qui présente des cavités ayant une nervure (n=1) et un deuxième rouleau de moulage qui présente des cavités sans nervure (n'=0).

4. Procédé selon la revendication 2, caractérisé par le fait qu'on utilise un premier rouleau de moulage qui présente des cavités ayant deux nervures (n=2) et un deuxième rouleau de moulage qui présente des cavités ayant une nervure (n'=1).

5. Procédé selon la revendication 1, caractérisé par le fait qu'on utilise un premier rouleau de moulage, qui présente des cavités ayant au moins une nervure (n ≥ 1) et on utilise, comme deuxième rouleau de moulage, un rouleau lisse.

6. Procédé selon l'une des revendications précédentes, caractérisé par le fait qu'on introduit dans les rouleaux de moulage le produit fondu contenant un principe actif entre deux feuilles constituées d'un matériau d'enveloppement, si bien qu'on obtient des comprimés en film ou bien des comprimés scellés dans un emballage à bulles.

7. Procédé selon l'une des revendications précédentes, caractérisé par le fait qu'on utilise des rouleaux de moulage ayant des cavités allongées ou du genre ellipsoïde, si bien qu'on obtient des comprimés oblongs, subdivisables, ou des comprimés à forme lenticulaire.

8. Dispositif de mise en oeuvre du procédé selon les revendications 1 à 7, comportant deux rouleaux de moulage (6, 8) pouvant tourner en sens inverses, présentant des cavités (2) destinées à recevoir et mouler le produit en fusion pour former des comprimés, caractérisé par le fait qu'au moins l'un des rouleaux de moulage présente des cavités (2) qui sont subdivisées au moyen d'au moins une nervure (7) qui s'étend sensiblement jusqu'à la surface d'enveloppe du rouleau de moulage et provoque le moulage d'une cannelure de rupture.

9. Dispositif selon la revendication 8, caractérisé par le fait que le premier rouleau de moulage (6) présente des cavités (2) qui sont subdivisées par n nervures et le deuxième rouleau de moulage (8) présente des cavités (2) correspondantes, qui sont subdivisées par n' nervures, n' étant situé dans la plage de 0 à n-1.

10. Dispositif selon la revendication 9, caractérisé par le fait que le premier rouleau de moulage (6) présente des cavités (2) ayant une nervure (7) (n=1) et le deuxième rouleau de moulage (8) présente des cavités (2) correspondantes sans nervures (n'=0).

11. Dispositif selon la revendication 9, caractérisé par le fait que le premier rouleau de moulage (6) présente des cavités (2) ayant deux nervures (7) (n=2) et le deuxième rouleau de moulage (8) présente des cavités (2) correspondantes ayant une nervure (7).

12. Dispositif selon la revendication 8, caractérisé par le fait que le premier rouleau de moulage (6) présente des cavités (2) ayant au moins une nervure (7) (n ≥ 1) et le deuxième rouleau de moulage est un rouleau lisse (8).

13. Dispositif selon l'une des revendications 8 à 12, caractérisé par le fait que l'on utilise des rouleaux de moulage (6, 8) ayant des cavités (2) allongées ou du genre de segment d'ellispoïde.
